# EUROPEAN PATENT SPECIFICATION

(11) **EP 2 991 566 B1**
(45) Date of publication and mention of the grant of the patent: **19.07.2017**
(21) Application number: 13725504.8
(22) Date of filing: 30.04.2013
(51) Int. Cl.: A61B 17/34, A61B 17/70, A61B 17/88

(54) **BONE CEMENT DELIVERY ASSEMBLY WITH LEAKAGE PREVENTION VALVE**
KNOCHENZEMENTFREISETZUNGSANORDNUNG MIT LECKSCHUTZVENTIL
ENSEMBLE DE DISTRIBUTION DE CIMENT ACRYLIQUE DOTÉ D'UNE VALVE DE PRÉVENTION DE FUITE

(43) Date of publication of application: 09.03.2016
(73) Proprietor: Stryker Corporation, Kalamazoo, MI 49002 (US)
(72) Inventor: BOBOLTZ, David, R., Kalamazoo, MI 49048 (US)
(74) Representative: Röthinger, Rainer
(86) International application number: PCT/US2013/038757
(87) International publication number: WO 2014/178833

(56) References cited:
- WO-A2-2007/140315
- US-A1- 2003 105 469
- US-A1- 2003 167 040

## Description

### Background Of The Invention

This invention relates generally to an assembly for injecting material into living tissue. One such material that can be injected using this assembly is bone cement. More particularly, the assembly of this invention is designed so that, when the delivery cannula integral with the assembly is removed and a new delivery cannula inserted, there is essentially no fluid leakage.

### Background Of The Invention

There are a number of different medical conditions in which the course of treatment involves the injection of cement into the hard tissue, the bone of the patient. One such procedure is a vertebroplasty procedure. In a vertebroplasty procedure bone cement is injected into a vertebra that was previously fractured. The procedure is performed to stabilize fractured vertebra. The procedure is performed to reduce the undesirable effects of the fracture. These effects are known to include, back pain, spinal deformity and loss of patient height.

In a vertebroplasty procedure and other procedures in which cement is injected into bone an assembly that consists of at least two cannulae is often employed to inject the cement. The first one of the cannula is an access cannula. The access cannula is used to define a portal from the outside environment, through soft tissue that surrounds the bone and into the bone into which the cement is to be injected. The assembly includes one additional cannula, referred to as a delivery cannula or cement cannula. The delivery cannula is dimensioned to be inserted into the lumen of the access cannula.

To actually deliver the bone cement the access cannula is first inserted into the patient. Often during the positioning process a pointed tip stylet is seated in the lumen of the access cannula. The tip of the stylet is the component of the assembly that pierces the tissue through which the cannula is inserted. The access cannula is positioned so the end of the cannula is positioned at the location in bone in which the cement is to be introduced. In some bone cement procedures a device is used to widen out space around the distal end of the access cannula. This is step is performed to ensure the presence of a void space into which the cement can flow. A previously filled delivery cannula is then seated in the access cannula. An obturator, a type of a plunger is then employed to force the bone cement out of the delivery cannula into the space into which the cement is to be introduced.

To minimize the trauma to the patient and to facilitate the precise control of the discharge of bone cement, both the access cannula and delivery cannula are relatively small in size. For example an access cannula often has an outer diameter of 5 mm or less. By extension that means that the inner diameter of the delivery cannula, the diameter of the lumen internal to the delivery cannula is 3.5 mm or less. This means that the volume of cement contained in any given filed delivery cannula is often 2 cm³ or less. Often a procedure in which it is necessary to inject bone cement into a patient requires a volume of cement that is greater than the volume contained in any one delivery cannula. This is why at the start procedure the practitioner has available two or more delivery cannula. Once the cement is discharged from one delivery cannula, the practitioner withdraws that cannula from the access cannula. A replacement delivery cannula filled with cement is then inserted into the cannula. Once the newly filled delivery cannula is in place, the part of the procedure in which the cement is actually injected into the bone can continue.

A problem during the cement injection procedure has been known to occur during the time period between the withdrawal of one delivery cannula from the access cannula and the insertion of a second access cannula. Specifically, during this time period, the access cannula, which is then empty, is known to fill with blood. This fluid is known to leak out of the proximal back end of the access cannula. To prevent this fluid leakage and the subsequent need to clean this fluid, the current practice is for the practitioner to, as soon as he/she removed the withdraws a delivery cannula, place a thumb or finger over the open proximal end of the cannula. This digit must be carefully positioned so as to not cause the movement of the access cannula. Having to so hold the access cannula while putting away the empty delivery cannula and fitting a filled delivery cannula back into the access tube can make the removal and refitting of these cannulae an ergonomically awkward experience.

Document US 2003/167040 A1 discloses a medical instrument with a hollow instrument canal that is configured inside a housing and can be closed by means of at least one valve body and rotated into open position by means of an instrument inserted into the instrument canal. The at least one valve body is configured as a flap consisting of an essentially non-bendable material mounted on the housing so that it can be rotated by means of an elastic, bendable connecting element configured as a wire spring in such a way that the valve body is pretensioned in the closed direction.

Document US 2003/105469 A1 relates to bioresorbable inflatable devices and tunnel incision tool and methods for treating and enlarging, e.g., a tissue. The device is composed of a hollow expanding pouch made of a resorbable material or a perforated material that can be attached to a filling element. The pouch can be filled with biocompatible materials, one or more times in few days interval, after the insertion of the device. The tunnel incision tool is composed of a little blade that emerges from the surface of the tool in order to make shallow incisions in the surrounding tissue therefore enabling easy expansion of the tissue.

Further, document WO 2007140315 A2 discloses a system for forming an implant to stabilize an interior of a vertebral body. The system includes a delivery cannula in which there is an agglomeration of beads and cement. A membrane is disposed over the open end of the cannula. When the agglomeration is discharged from the cannula it fills the membrane. The membrane thus forms the outer shell of the implant internal to the bone.

### Summary Of The Invention

This invention relates to a new and useful assembly for injecting a substance such as bone cement into a living being. This assembly typical includes both an access cannula and a delivery cannula. The assembly of this invention is designed to minimize the leakage of fluid from the assembly during time periods when another component of the assembly is not disposed in the access cannula.

The access cannula of this invention includes a valve. Specifically, the valve of this invention allowed
insertion and withdrawal of a delivery cannula. When a delivery cannula or other device is not seated in the access cannula, the valve blocks the flow of fluid out of the access cannula.

In many versions of this invention the valve and access cannula are further constructed to apply a restraining force on the delivery cannula when the delivery cannula is withdrawn from the access cannula. This restraining forcing substantially eliminates the likelihood that the presence of a pressure head at the site to which the assembly is applied can force the delivery cannula out of the access cannula.

In some versions of the invention, the valve has one or more valve flaps formed from elastomeric material. In some alternative versions of the invention, the valve may include another valve element formed from elastomeric material. Alternatively, the valve may have a biasing spring or springs that hold the valve flaps in the closed state.

A more specific version of this invention is used to deliver bone cement into a bone such as a vertebral body. This version of the invention includes a bone cement mixing assembly. As the name implies, this assembly produced bone cement. The delivery cannula of this version of this invention, sometimes referred to as a delivery cannula, is adapted to be releasably secured to an outlet fitting of the mixing assembly. Once the bone cement is prepared the cement is loaded into the delivery cannula. The delivery cannula is then fitted into the positioned access cannula. An obturator is inserted through the delivery cannula to push the cement out into the space internal to the bone at which the cement is to be delivered.

Some assemblies of this invention do not include the delivery cannula. In these versions of the invention, the valve internal to the access cannula is again normally closed. In these versions of the invention, the insertion of a stylet, the injection of cement or the insertion of an obturator into the lumen of the access cannula provides sufficient force to overcome the force of the valve that holds the valve closed. The withdrawal of either the stylet or obturator, or terminating of the force applied to the cement for injection purposes results in the valve closing. The pressure head of fluid that flows proximally from the open distal end of the access cannula does not provide sufficient force to urge the valve into the open state.

### Brief Description Of The Drawings

The invention is pointed out with particularity in the claims. The above and further features and benefits of this invention are understood from the following Detailed Description taken in conjunction with the following drawings in which:
Figure 1 is an exploded view of the components of the delivery system of this invention;
Figure 2 is an exploded view of the components forming the access cannula;
Figure 3 is a cross sectional view of the proximal end of the access cannula;
Figure 4 is a plan view looking distally forward of the hub and valve of the access cannula;
Figure 5 is an exploded view depicting how the delivery cannula of this assembly, sometimes referred to as a delivery cannula, is attached to a bone cement mixer;
Figure 6 is a perspective view of the insertion of the delivery cannula, sometimes specifically referred to as the delivery cannula, inserted into the access cannula;
Figure 7 is a cross sectional view of an alternative delivery assembly of this invention;
Figure 7A is an enlarged view of a portion of the cross sectional view of Figure 7; and
Figure 8 depicts an alternative cement mixer and cement delivery assembly of this invention.

### Detailed Description

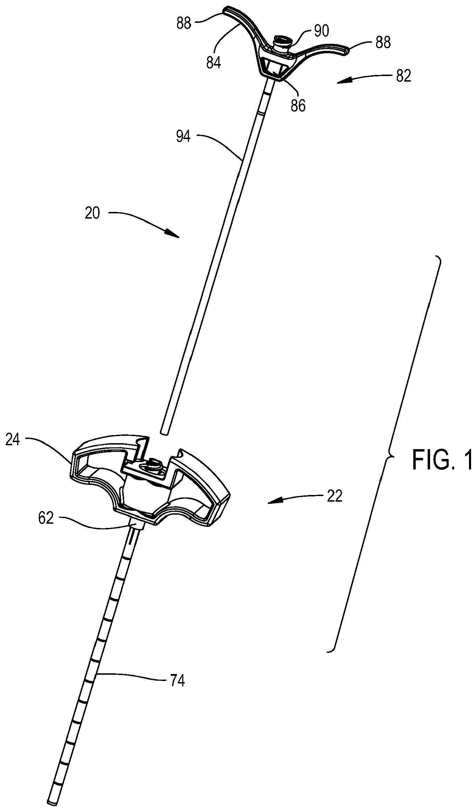

Figure 1 illustrates a delivery assembly 20 of this invention useful for introduce fluid and semisolids into living tissue. Often assembly 20 of this invention is employed to deliver bone cement. Assembly 20 includes an access cannula 22 and a delivery cannula 82. The access cannula 22 is used to form a portal that leads to the location internal to the patient at which the material is to be introduced. The delivery cannula 82 is dimensioned to be slidably and removably disposed in the access cannula 22. Prior to insertion of the delivery cannula 82 into the access cannula 22, the delivery cannula is filled with the material to be introduced into the tissue. Once the delivery cannula 82 is seated in the access cannula 22 an obturator is used to force the material out of the delivery cannula into the tissue.

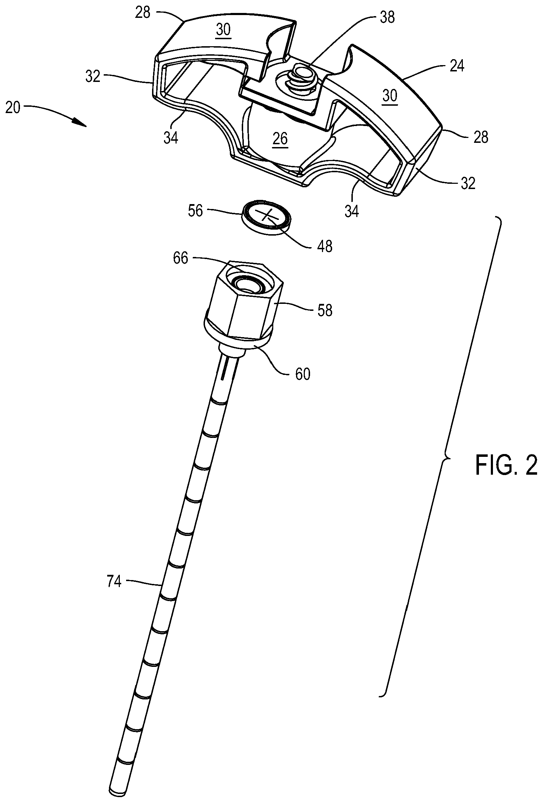

Access cannula 22, as seen best in Figures 2-4, includes a handle 24. Handle 24 is a single piece unit that includes a base 26. In the depicted version of the invention, base 26 has a cylindrical outer wall. Two finger grips 28 extend outwardly from the opposed sides of the base 26. Each finger grip 28 includes a beam 30 that extend outwardly from the base 26 adjacent the proximal end of the base. (Here "proximal" is understood to mean towards the practitioner holding the access cannula 22, away from the tissue to which the cannula is to be directed. "Distal" is understood to mean away from the practitioner, towards the tissue to which the cannula 22 is to be directed.) A beam 34, located below the beam 30, extends outwardly away from the base 26 adjacent the distal end of the base. A web 32 extends between the free ends of the beams 30 and 34.

A fitting 38 with threading (not identified) extends upwardly from the center of proximally directed face of handle base 26. Fitting 38 has a lumen 40. Handle 24 is formed so that lumen 40 extends a short distance into handle base 26. Fitting lumen 40 opens into bores 41 and 42 that are coaxial with the lumen and within the handle base 26. Bore 41 has a diameter approximately 2 mm greater than that of lumen 40. Bore 42 has a diameter approximately 9 mm greater than that over bore 41. In terms of overall length, bore 42 extends at least 50% of the distance through the handle base 26. In some versions of the invention, bore 42 is in the form of a polygon. In still more particular versions of the invention, bore 42 is in the form of a hexagon. A counterbore 44, coaxial with bore 42, forms the open distal end of handle base 26. Counterbore 44 is circular in cross section.

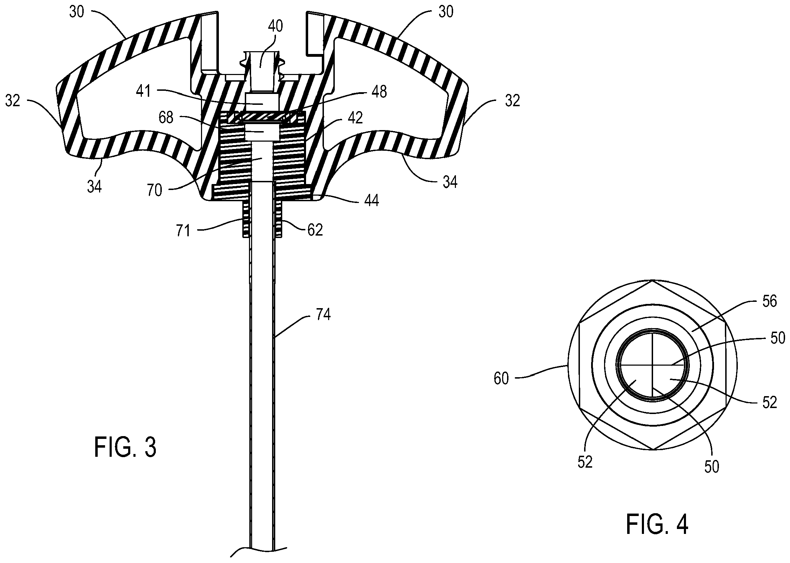

A valve 48 is seated in the proximal end base of bore 42, where the fitting bore 41 opens into bore 42. Valve 48 as seen best in Figures 2 and 4, is the form a disc shaped piece of rubber. The valve 48 has a thickness of between 0.5 and 3.0 mm. Valve 48 is formed to have two slits 50 that are perpendicular to each other and that cross at the center of the valve. Slits 50 define valve flaps 52, only two flaps identified.

In the illustrated version of the invention, valve 48 is encased in a ring 56. Ring 56 is often formed from nylon or other plastic and has a wall thickness of between 1 and 4 mm. In some methods of manufacturing assembly 20, after ring 56 is molded, valve 44 is molded within the ring 52. Once the valve 44 is formed, the flap-defining slits 46 are formed.

Also disposed in handle bore 42 is a hub 58 that is formed from nylon or other plastic. Hub 58 has a body, (not identified) that has a cross sectional shape that allows the hub to be press fit in bore 42. In the depicted version of the invention, the hub body has a shape in cross section that is hexagonal. At the distal of the body a rim 60 extends radially outwardly from the hub body. Rim 60 is designed to press fit in handle counterbore 44. A sleeve 62 extends outwardly from the distally directed face of the hub. Hub 58 has a length such that when the hub is mounted to handle 24, the distally directed face of the hub is flush with the handle and sleeve 62 extends forward from the handle.

A number of bores form a through channel that extends longitudinally through hub 58 along the longitudinal axis through the hub. A first bore, bore 66, extends distally from the proximal end of hub 58. Bore 66 has a diameter that facilitates the press fitting of ring 56 in the bore 66. Bore 66 has a depth such that when the ring 56 is disposed in the bore, the ring is flush with the proximally directed face of hub 58. Bore 66 opens into a bore 68. Bore 68 has a diameter that is less than the diameter of bore 66. In many versions of the invention hub bore 68 has the same diameter as handle base bore 41. Bore 68 opens up into a bore 70. Bore 70 is smaller in diameter than bore 68. Bore 70 opens up into a bore 71. Bore 71 is the distalmost bore of the hub 58. Bore 71 forms the lumen through sleeve 62.

A tube 74, that forms the actual body of the access cannula 22, extends proximally from the hub 58. Tube 74, being the body of the access cannula 22, is the component of the cannula 22 that is inserted into the patient. The tube 74 is formed from stainless steel or other material capable of being inserted into living tissue. Tube 74 has an outer diameter of typically 5 mm or less and often 3.5 mm or less. Tube 74 has a wall thickness of 0.5 mm. Tube 74 is seated in hub bore 71 and extends through and forward of sleeve 62.

In some versions In some methods of manufacturing the access cannula 22, hub 58 is overmolded over tube 74. This overmolded thus forms hub bore 71. Once the hub and tube sub-assembly is formed, the ring and valve assembly is press fit in bore 66. Hub 58 with the components attached thereto, is then press fit in handle bore 42. The process of fitting the hub 58 and attached components to the handle 24.

Returning to Figure 1, it is seen that the delivery cannula 82 includes a handle 84. The handle 84 includes a base 86 from which two opposed wings 88 extend. A fitting 90 extends proximally outwardly from the proximal face of base 86. Fitting 90 is formed with features that facilitate the attachment of fitting to a mixer in which the cement is held prior to being loaded in the delivery cannula.

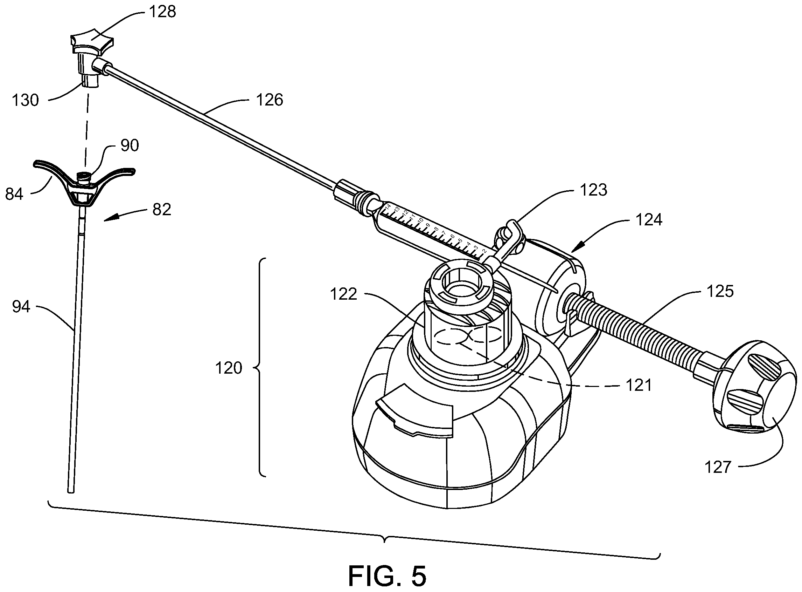

One such cement mixer 120 that can be incorporated into this invention is seen in Figure 5. Mixer 120 includes a first chamber 122 in which the material to be filled in the cannula is mixed. Not seen is the paddle disposed in chamber 122 that mixes the components forming the cement together to form the cement. Mixer 120 also includes a second chamber 124 into which the material is initially flowed after mixture. Not identified is the tube that extends from the first chamber 122 to the second chamber through which the mixed cement is flowed to the second chamber 124. An extension tube 126 extends from the second chamber 124. An outlet fitting 130 is attached to the distal end of the extension tube 126. Mixer outlet fitting 130 has the features that engage the fitting 90 integral with the delivery cannula 82. These features are configured to facilitate the releasable engagement of the delivery cannula to the mixer 120. In the depicted version of the invention a valve 128 controls fluid flow through mixer outlet fitting 128. The Applicant's US Patents No. 6,547,432 issued 15 April 2003, and 7,658,537 issued 9 February 2012, each of which is incorporated herein by reference, provide further description of the construction of cement mixers. It should be understood that the exact structure cement mixer that may be integrated into this invention is not part of the present invention.

Returning to Figure 1 it can be understood that delivery cannula 82 includes a tube 94 that extends distally forward from handle 84. Tube 94 has an outer diameter that allows the tube 94 to slip fitting in access cannula fitting 38 and the lumen of access cannula tube 74. In many versions of the invention, the lumen internal to tube 94 (lumen not identified) has a volume of 2 cm³ or less, typically 1.5 cm³ or less and sometimes 1.0 cm³ or less.

In many versions of the invention, handle 84 is overmolded over tube 94 to form the delivery cannula 82.

Delivery assembly 20 of this invention is prepared for use by filing the tubes 94 of one or more delivery cannulae 82 with the material that is to be introduced into the living tissue. Figure 5 depicts the filling of a single delivery cannula 82 from cement mixer 120. When assembly 20 of this invention is used to inject bone cement into tissue, the delivery cannula 82 is sometimes referred as a cement cannula.

A stylet is fitted in the access cannula 22. The tip of the stylet extends forward of the distal end of cannula tube 74. The access cannula and stylet are inserted into the patient. The access cannula 22 is positioned so that distal end of the cannula is positioned adjacent the location where the fluid or semi-solid to be introduced. The stylet is removed. A void creation tool, such as a balloon may then be inserted in the cannula. This tool, which is not part of the present invention, is used to create a void space into which the fluid, semi-solid or other material to be introduced. The device is withdrawn from the cannula.

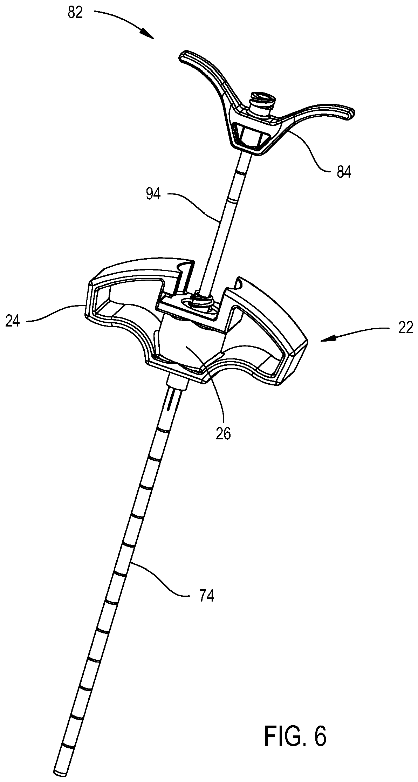

A delivery cannula is then inserted in the access cannula as seen in Figure 6. More specifically the delivery cannula 82 is positioned so that delivery cannula tube 94 seats in access cannula tube 74. An obturator or other plunger like device is then inserted in the access cannula tube 84. This device forces the material in the delivery cannula tube 94 out of the cannula and into the tissue in which the material will have a therapeutic benefit.

During period of time in which there is neither a void-creating device nor a delivery cannula 82 in the access cannula 24, the flaps 52 of valve 48, abut. The flap-against flap contact blocks the flow of fluid present in the empty access cannula tube 74 out through the cannula fitting 38. When a stylet, a void creation tool or a delivery cannula 82 is inserted in the access cannula, the flaps are flexed downwardly, into the outer annular portion of hub bore 68. Thus the presence of the flaps does not impede the insertion of the device inserted into the access cannula through either the hub 58 or cannula tube 74.

The valve flaps are formed from elastomeric material, material that, upon the removal of a deforming force, returns to its undeformed state. Accordingly once the device is removed from the access cannula 22, the valve flaps 52 flex back to the abutting, closed valve, state. Valve 44 prevents flow of liquid that may enter access cannula tube 74 from percolating up and out of the access cannula fitting 38. Thus, during time periods when there is neither a stylet, a void creation tool or a delivery cannula disposed in the access cannula, the assembly of this invention inhibits the unwanted discharge of fluid out of the access cannula 22. This means that during these time periods of procedure, neither the practitioner nor an assistant need to hold a thumb or finger over access cannula fitting 38 to prevent such discharge. This frees the individual having to perform this task so he/she can concentrate on other aspects of the procedure.

It is a further feature of the assembly 20 of this invention that the valve 44 automatically opens upon the insertion of the complementary device in the access cannula 22. Valve 44 likewise automatically closes upon withdrawal of the device from the access cannula 22. This means that the assembly of this invention eliminates the undesirable discharge of fluid without requiring the medical personnel to take any additional steps.

The assembly of this invention does more than just prevent fluid flow out of the access cannula when a first delivery cannula is withdrawn and a new delivery cannula put in position. The valve prevents blood loss out of the access cannula in the time period between when the void creation tool is withdrawn from the access cannula and a delivery cannula inserted into the access cannula. In practice it has been found that in a procedure in which two access cannulae are simultaneously inserted into a patient, the creation of void space adjacent a first one of the cannula results in a build up of pressure adjacent the distal end of the second access cannula. This pressure build up forces fluid, primarily blood, through the access cannula 22. If neither a stylet, a void creation tool nor a delivery cannula is disposed in the second access cannula, the valve limits, if not blocks the flow of this fluid out of the proximal end of the second access cannula.

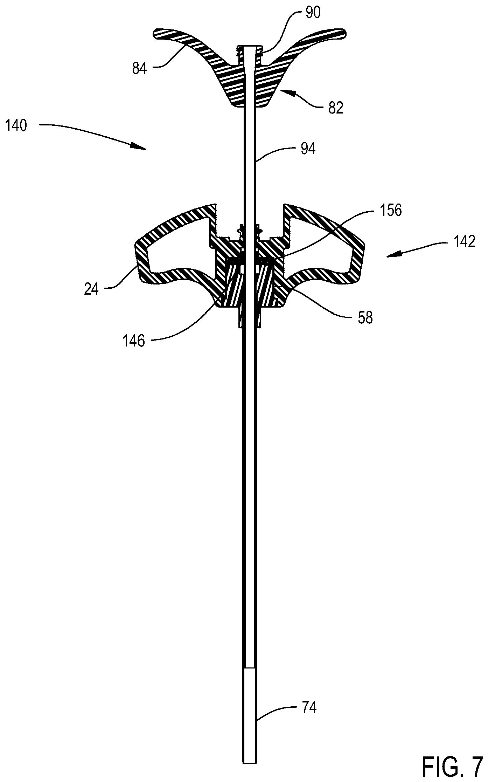

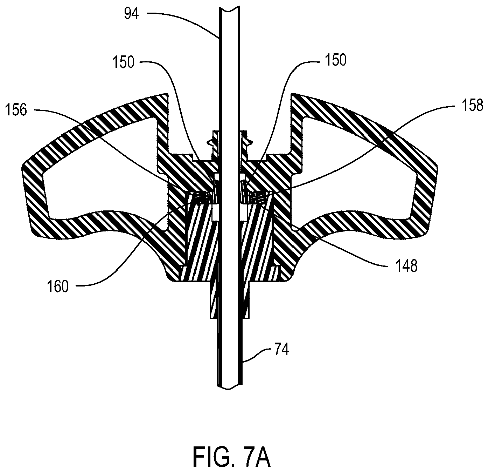

Figures 7 and 7A illustrate an alternative delivery assembly 140 of this invention. Delivery assembly includes access cannula 142 and the previously described delivery cannula 82. Many of the components of access cannula 142 are similar to those of the first described access cannula 22 accordingly these components will not be redescribed.

Instead of valve 48 and ring 56, access cannula 142 includes a valve 146 and a ring 156. Valve 146 is formed from the same material from which valve 48 is formed. Valve 48 is disc shaped. The valve 146 is further formed to have a groove 148 that extends inwardly from the circular side surface of the valve. Groove 148 extends circumferentially around the valve 146. While not identified, valve 146 is formed with slits similar to slits 50 of valve 48. These slits separate the center portion of the valve 146 into flexible flaps 150.

In this version of the invention, the components forming the assembly 140 are constructed so that the radius of delivery cannula tube 94 and the thickness of a valve flap 150 is approximately 0.5 mm greater than the radius of bore 41 internal to access cannula fitting 38. The reason for this dimensioning is apparent below.

Ring 156 is formed from the same material from which ring 56 is formed. Ring 156 has a circular main body 158 similar to the body of ring 56. The ring 156 is further formed to have a lip 160 that extends inwardly from the cylindrically shaped inner surface of the main body 160. Ring 156 is shaped so lip 160 can seat in groove 148 internal to valve 146. Again in some versions of the invention, valve 146 is molded into ring 156.

Valve 146 and ring 156 are seated in the proximal end base of bore 42. More particularly, the valve 146 and ring 156 are seated in bore 66 internal to hub 58.

Delivery assembly 160 functions in the same general manner in which the first described delivery assembly 20 functions. Unless a stylet or a delivery cannula 82 is seated in access cannula 142, valve flaps 150 abut so as to place the valve in the closed state. Valve 146 thus prevents the flow of material out of the access cannula. The insertion of the stylet or delivery cannula 52 into the cannula handle 24 provides sufficient force to flex the valve flaps 150 inwardly. This flexing of the valve flaps opens the valve 146.

When the delivery cannula 82 is disposed in the access cannula there may be times when a head of pressure develops at the site at which the distal end of the assembly 160 is located. This pressure may urge delivery cannula 82 proximally outwardly. If this event occurs, the valve flaps 150 move, flex proximally, with the cannula. The valve flaps flex into the annular space in fitting bore 41 around the delivery cannula 82. Owing to the dimensions of the components, the valve flaps 150 are compressed against the tube 94 integral with the delivery cannula 82. This compressive force is typically greater than force of the fluid that is pushing the delivery cannula 82 outwardly. Thus valve 150 of this version of the invention, further functions as a brake that stops pressure generated at the site to which the assembly is applied from forcing the delivery cannula 82 out of the access cannula when such movement is not desired.

The manual force a practitioner applies to the delivery cannula 82 to withdraw the delivery cannula from the access cannula 142 is sufficient to overcoming the braking force the valve applies to the cannula tube 94 to inhibit unintended retraction of the delivery cannula 82.

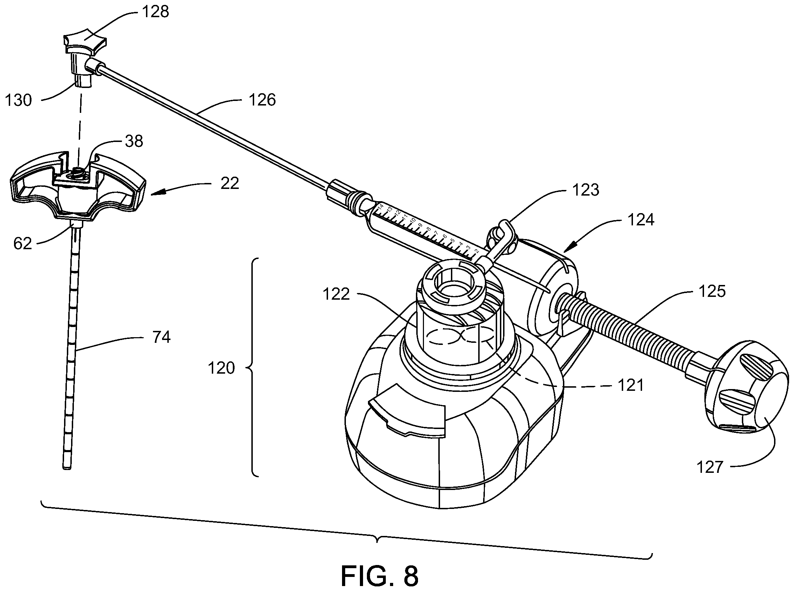

Figure 8 illustrates another assembly of this invention. Here access cannula 22 is connected directly to cement mixer 120. In this version of the invention, access cannula fitting 38 is adapted to be releasably attached to mixer outlet fitting 130. Thus this version of the assembly of this invention does not include a delivery cannula. In this version of the invention, the access cannula is typically attached filled with cement after the cannula is positioned adjacent the tissue into which the cement is to be flowed.

In this version of the invention, valve 48 (Figure 2) is still normally closed. The valve 48 prevents back flow of fluid out of the access cannula when neither the stylet nor obturator are fitted in the cannula 22. It should be appreciated that in this version of the pressure applied when the cement is forced through the cement mixer by piston 125, is sufficient to open up the valve. When this pressure is released, the elastomeric force of the valve returns the valve to the closed state.

It should be understood that the foregoing is directed to specific versions of the delivery assembly of this invention. Other versions of the assembly may have features different from what has been described.

For example, it should be clear from the text, that the assembly of this invention can be employed to deliver fluids and semi-solids into living tissue other than bone cement. Generally this means that cannula 82, the cannula disposed in the access cannula can be called a delivery cannula. Thus the structure of the fitting of the delivery cannula 82 may be specific to the fill device employed to load the cannula with the material to be injected into the tissue.

The structure of the handles integral with cannulae 22 and 82 is understood to be exemplary, not limiting. Likewise, some access cannulae and delivery cannulae of this invention may have additional components that, when employed create the void space into which the material that is inject is to be injected.

Further, this invention is not limited to valves wherein the moving valve elements are one or more elastomeric flaps. In some versions of the invention, the valve may be a mechanical valve. This valve for example could be a flapper valve. A spring normally holds the valve element closed. When a stylet, a void creation tool or a delivery cannula is inserted in the access cannula the force of the insertion overcomes the spring force so as to pivot the valve open. The actual valve element would pivot distally to an open. Another valve of this invention may be a duck-billed valve.

Other elastomeric devices than the disclosed planar member may alternatively function as the valve elements of the valve. In one such assembly, the valve element is planar. The valve element is formed with a relatively small opening that is normally closed. The insertion of stylet, a delivery cannula or an obturator into the access cannula provides enough force against the valve to force the dilation of the valve so that the opening expands in diameter from the closed state to an open state. In still other versions of the invention, the valve is toroidal in shape. The inner surfaces of the material forming the valve normally abut so as to hold the valve in the closed shape. The force of the stylet, the delivery cannula or obturator against the inner surface of the valve element causes the radially outward compression of the valve element. In other words, the valve element is dilated open. In both these versions of the invention the pressure head of the fluid that may be directed proximally within the access cannula is not sufficient to force the valve element into the open state.

Dimensions, unless recited in the claims, are understood to be exemplary, not limiting.

Accordingly, it is an object of the appended claims to cover all such variations and modifications of the invention.

## Claims

1. A delivery assembly (20, 140) for delivering material into living tissue, the delivery assembly including:
an access cannula (22, 142) that includes a tube (74) formed from material capable of being inserted into a living body, the tube having opposed proximal and distal ends, the distal end of the tube configured for insertion into the living body and a lumen that extends through the tube;
a delivery cannula (82) the delivery cannula having a tube (94) with opposed proximal and distal ends, the delivery cannula tube dimensioned to seat in the lumen of the access cannula tube (74) and a fitting (90) that leads to the distal end of the access cannula tube, the fitting dimensioned to receive a connector (130) in communication with a container (124) in which the material to be delivered into the living tissue is contained,
**characterized in that**:
a valve (48, 146) is attached to the access cannula adjacent the proximal end of the access cannula tube, said valve having at least one element (52, 150) that is normally in a closed position over the access cannula tube (74) to prevent the proximal flow of material out of the tube and that is capable of moving to an open position upon the manual insertion of the delivery cannula tube (94) in the access cannula (22, 142) and a biasing member that, upon removal to the delivery cannula tube from the access cannula tube, returns the at least one element to the closed position.

2. The delivery assembly (140) of Claim 1, wherein said valve (146) is further configured to, during removal of the delivery cannula tube (94) from the access cannula tube (74), press said at least one valve element (150) against the delivery cannula tube (94) to inhibit retraction of the delivery cannula tube from the access cannula tube.

3. The delivery assembly (140) of Claim 1, wherein the access cannula (22), the delivery cannula (82) and said valve (146) are collectively configured to, during removal of the delivery cannula tube (94) from the access cannula tube (74), compress said at least one valve element (150) between an interior surface of the access cannula and the delivery cannula tube.

4. The delivery assembly (20, 140) of any one of Claims 1 to 3, wherein:
the access cannula (22) includes a handle (24) to which the proximal end of the access cannula tube (74) is mounted and from which the access cannula tube extends distally forward, said handle including at least one bore (41, 42) that extends to the access cannula tube; and
said valve (48, 146) is mounted to said access cannula handle and is positioned so that when said at least one valve element (52, 150) moves between the closed and open positions, said at least one valve element is disposed only within said handle.

5. The delivery assembly (20, 140) of Claim 4, wherein:
said valve (48, 146) is mounted in the access cannula handle (24) so as to be between a proximal bore (41) and a distal bore (68), the handle proximal bore (41) and the handle distal bore (68) each having a diameter greater than the lumen of the access cannula tube (74);
during insertion of the delivery cannula tube (94) into the access cannula tube (74) the at least one valve element (52) pivots into the access cannula handle distal bore (68);
during removal of the delivery cannula tube (94) from the access cannula tube (74), the at least one valve element pivots into the access cannula handle proximal bore (41).

6. The delivery assembly (20, 140) of any one of Clams 1 to 5, wherein said at least one valve element (52, 150) is formed from elastomeric material so that said valve element functions as said biasing member that urges said valve element into the closed position.

7. The delivery assembly (20, 140) of any one of Claims 1 to 6, wherein said valve (48, 146) is a planar member formed from elastomeric material that has at least one slit, the slit defining at least one flap (52, 150), said flap being the at least one valve element.

8. The delivery assembly (20, 140) of Claim 7, wherein said valve (48, 146) is formed with plural slits so as to define plural said valve flaps (52, 150).

9. The delivery assembly of Claims 7 or 8, wherein said valve (150) is disc shaped.

10. The delivery assembly (20, 140) of Claims 7, 8 or 9, wherein said valve (48, 146) is disposed in a ring (56, 156) that circumferentially surrounds said valve.

11. The delivery assembly (20, 140) of any one of Claims 1 to 10, wherein said valve (48, 146) includes plural said valve elements (52, 150).

12. The delivery assembly of Claim 11, wherein said valve (48, 146) includes four said valve elements (52, 150).

13. The delivery assembly (20, 140) of any one of Claims 1 to 12, wherein said delivery cannula includes a fitting (90) shaped to receive an complementary outlet fitting (130) from a bone cement mixing and delivery device.

14. A system for delivering bone cement comprising:
a bone cement mixing assembly (120), the bone cement mixing assembly having a first chamber (122) in which the bone cement is mixed and an outlet fitting (130) in fluid communication with the first chamber from which the mixed bone cement is delivered;
**characterized in that** the system further comprises:
the delivery assembly (20, 140) of any one of Claims 1 to 13 wherein the delivery cannula fitting (90) is connected to the outlet fitting (130) of the bone cement mixing and delivering assembly for receiving the mixed bone cement.

15. The system for delivering bone cement of Claim 14, wherein: the bone cement mixing assembly (120) further includes a second chamber (124) that is connected to the first chamber (122) to which the cement is flowed after mixing; and said bone cement mixing assembly outlet fitting (130) is connected to said second chamber (124) to received mixed bone cement from the second chamber.

## Patentansprüche

1. Zuführanordnung (20, 140) zum Zuführen von Material in lebendes Gewebe, wobei die Zuführanordnung umfasst:
eine Zugangskanüle (22, 142), die umfasst: eine aus einem in einen lebenden Körper einführbaren Material gebildete Röhre (74), wobei die Röhre entgegengesetzte proximale und distale Enden aufweist, und wobei das distale Ende der Röhre zum Einführen in den lebenden Körper ausgebildet ist, und ein Lumen, das sich durch die Röhre erstreckt;
eine Zuführkanüle (82), wobei die Zuführkanüle aufweist: eine Röhre (94) mit entgegengesetzten proximalen und distalen Enden, wobei die Zuführkanüle so dimensioniert ist, dass sie in dem Lumen der Zugangskanülenröhre (74) sitzt, und ein Anschlussstück (90), das zu dem distalen Ende der Zugangskanülenröhre führt, wobei das Anschlussstück so dimensioniert ist, dass es ein Verbindungsstück (130) aufnimmt, das mit einem Behälter (124) verbunden ist, in dem das in das lebende Gewebe zuzuführende Material enthalten ist,
**dadurch gekennzeichnet, dass**:
ein Ventil (48, 146) an der Zugangskanüle neben dem proximalen Ende der Zugangskanülenröhre angebracht ist, wobei das Ventil aufweist: wenigstens ein Element (52, 150), das sich im Normalzustand in einer geschlossenen Position über der Zugangskanülenröhre (74) befindet, um den proximalen Materialfluss aus der Röhre heraus zu verhindern, und das auf ein manuelles Einbringen der Zuführkanülenröhre (94) in die Zugangskanüle (22, 142) hin in eine geöffnete Position bewegbar ist, und eine Vorspannkomponente, die auf ein Entfernen der Zuführkanülenröhre von der Zugangskanülenröhre hin das wenigstens eine Element in die geschlossene Position zurückführt.

2. Zuführanordnung (140) nach Anspruch 1, wobei das Ventil (146) ferner dazu ausgebildet ist, während des Entfernens der Zuführkanülenröhre (94) von der Zugangskanülenröhre (74) das wenigstens eine Ventilelement (150) gegen die Zuführkanülenröhre (94) zu drücken, um ein Zurückziehen der Zuführkanülenröhre aus der Zugangskanülenröhre zu hemmen.

3. Zuführanordnung (140) nach Anspruch 1, wobei die Zugangskanüle (22), die Zuführkanüle (82) und das Ventil (146) gemeinsam dazu ausgebildet sind, während des Entfernens der Zuführkanülenröhre (94) von der Zugangskanülenröhre (74) das wenigstens eine Ventilelement (150) zwischen einer Innenfläche der Zugangskanüle und der Zuführkanülenröhre zusammenzudrücken.

4. Zuführanordnung (20, 140) nach einem der Ansprüche 1 bis 3, wobei:
die Zugangskanüle (22) einen Griff (24) umfasst, an dem das proximale Ende der Zugangskanülenröhre (74) angebracht ist und von dem aus sich die Zugangskanülenröhre distal vorwärts erstreckt, wobei der Griff wenigstens eine Bohrung (41, 42) umfasst, die sich zu der Zugangskanülenröhre erstreckt; und
das Ventil (48, 146) an dem Zugangskanülengriff angebracht ist und so angeordnet ist, dass, wenn sich das wenigstens eine Ventilelement (52, 150) zwischen den geschlossenen und geöffneten Positionen bewegt, das wenigstens eine Ventilelement nur in dem Griff angeordnet ist.

5. Zuführanordnung (20, 140) nach Anspruch 4, wobei:
das Ventil (48, 146) in dem Zugangskanülengriff (24) so angebracht ist, dass es sich zwischen einer proximalen Bohrung (41) und einer distalen Bohrung (68) befindet, wobei die proximale Bohrung (41) des Griffs und die distale Bohrung (68) des Griffs jeweils einen größeren Durchmesser als das Lumen der Zugangskanülenröhre (74) aufweisen;
während des Einbringens der Zuführkanülenröhre (94) in die Zugangskanülenröhre (74) das wenigstens eine Ventilelement (52) in die distale Bohrung (68) des Zugangskanülengriffs schwenkt;
während des Entfernens der Zuführkanülenröhre (94) von der Zugangskanülenröhre (74) das wenigstens eine Ventilelement in die proximale Bohrung (41) des Zugangskanülengriffs schwenkt.

6. Zuführanordnung (20, 140) nach einem der Ansprüche 1 bis 5, wobei das wenigstens eine Ventilelement (52, 150) aus einem elastomeren Material gebildet ist, so dass das Ventilelement als die Vorspannkomponente wirkt, die das Ventilelement in die geschlossene Position drückt.

7. Zuführanordnung (20, 140) nach einem der Ansprüche 1 bis 6, wobei das Ventil (48, 146) eine aus einem elastomeren Material gebildete flache Komponente ist, die wenigstens einen Schlitz aufweist, wobei der Schlitz wenigstens eine Lasche (52, 150) definiert, wobei die Lasche das wenigstens eine Ventilelement ist.

8. Zuführanordnung (20, 140) nach Anspruch 7, wobei das Ventil (48, 146) mit mehreren Schlitzen ausgebildet ist, um mehrere der Ventillaschen (52, 150) zu definieren.

9. Zuführanordnung nach Anspruch 7 oder 8, wobei das Ventil (150) scheibenförmig ist.

10. Zuführanordnung (20, 140) nach Anspruch 7, 8 oder 9, wobei das Ventil (48, 146) in einem Ring (56, 156) angeordnet ist, der das Ventil umfänglich umgibt.

11. Zuführanordnung (20, 140) nach einem der Ansprüche 1 bis 10, wobei das Ventil (48, 146) mehrere der Ventilelemente (52, 150) umfasst.

12. Zuführanordnung nach Anspruch 11, wobei das Ventil (48, 146) vier der Ventilelemente (52, 150) umfasst.

13. Zuführanordnung (20, 140) nach einem der Ansprüche 1 bis 12, wobei die Zuführkanüle ein Anschlussstück (90) umfasst, das dazu ausgebildet ist, ein komplementäres Ausgangsanschlussstück (130) einer Knochenzementmisch- und -zuführeinrichtung aufzunehmen.

14. System zum Zuführen von Knochenzement, das umfasst:
eine Knochenzementmischanordnung (120), wobei die Knochenzementmischanordnung aufweist: eine erste Kammer (122), in der der Knochenzement gemischt wird, und ein fluidisch mit der ersten Kammer verbundenes Ausgangsanschlussstück (130), von dem der gemischte Knochenzement zugeführt wird;
**dadurch gekennzeichnet, dass** das System ferner umfasst:
die Zuführanordnung (20, 140) nach einem der Ansprüche 1 bis 13, wobei das Anschlussstück (90) der Zuführkanüle mit dem Ausgangsanschlussstück (130) der Knochenzementmisch- und -zuführeinrichtung verbunden ist, um den gemischten Knochenzement aufzunehmen.

15. System zum Zuführen von Knochenzement nach Anspruch 14, wobei: die Knochenzementmischanordnung (120) ferner eine zweite Kammer (124) umfasst, die mit der ersten Kammer (122) verbunden ist, zu der der Zement nach dem Mischen fließt; und wobei das Ausgangsanschlussstück (130) der Knochenzementmischanordnung mit der zweiten Kammer (124) verbunden ist, um gemischten Knochenzement von der zweiten Kammer aufzunehmen.

## Revendications

1. Ensemble (20, 140) d'administration pour administrer un matériau dans un tissu vivant, l'ensemble d'administration incluant :
une canule (22, 142) d'accès qui inclut un tube (74) constitué d'un matériau apte à être inséré dans un corps vivant, le tube ayant des extrémités proximale et distale opposées, l'extrémité distale du tube configurée pour une insertion dans le corps vivant et une lumière qui s'étend à travers le tube ;
une canule (82) d'administration, la canule d'administration ayant un tube (94) avec des extrémités proximale et distale opposées, le tube de canule d'administration dimensionné de façon à loger dans la lumière du tube (74) de canule d'accès et un raccord (90) qui conduit jusqu'à l'extrémité distale du tube de canule d'accès, le raccord dimensionné pour recevoir un connecteur (130) en communication avec un récipient (124) dans lequel le matériau devant être administré dans le corps vivant est contenu,
**caractérisé en ce que** :
une valve (48, 146) est fixée à la canule d'accès adjacente à l'extrémité proximale du tube de canule d'accès, ladite valve ayant au moins un élément (52, 150) qui est normalement dans une position fermée par-dessus le tube (74) de canule d'accès pour empêcher l'écoulement proximal du matériau hors du tube et qui est apte à se déplacer jusqu'à une position ouverte à l'insertion manuelle du tube (94) de canule d'administration dans la canule (22, 142) d'accès et un élément de poussée qui, à l'enlèvement du tube de canule d'administration du tube de canule d'accès, ramène l'au moins un élément à la position fermée.

2. Ensemble (140) d'administration selon la revendication 1, dans lequel ladite valve (146) est en outre configurée pour, lors de l'enlèvement du tube (94) de canule d'administration du tube (74) de canule d'accès, presser ledit au moins un élément (150) de valve contre le tube (94) de canule d'administration pour empêcher un retrait du tube de canule d'administration du tube de canule d'accès.

3. Ensemble (140) d'administration selon la revendication 1, dans lequel la canule (22) d'accès, la canule (82) d'administration et ladite valve (146) sont collectivement configurées pour, lors de l'enlèvement du tube (94) de canule d'administration du tube (74) de canule d'accès, compresser ledit au moins un élément (150) de valve entre une surface intérieure de la canule d'accès et le tube de canule d'administration.

4. Ensemble (20, 140) d'administration selon l'une quelconque des revendications 1 à 3, dans lequel :
la canule (22) d'accès inclut une poignée (24) sur laquelle l'extrémité proximale du tube (74) de canule d'accès est montée et depuis laquelle le tube de canule d'accès s'étend distalement vers l'avant, ladite poignée incluant au moins un alésage (41, 42) qui s'étend jusqu'au tube de canule d'accès ; et
ladite valve (48, 146) est montée sur ladite poignée de canule d'accès et est positionnée de telle manière que, lorsque ledit au moins un élément (52, 150) de valve se déplace entre les positions fermée et ouverte, ledit au moins un élément de valve n'est disposé qu'à l'intérieur de la poignée.

5. Ensemble (20, 140) d'administration selon la revendication 4, dans lequel :
ladite valve (48, 146) est montée dans la poignée (24) de canule d'accès de manière à être entre un alésage proximal (41) et un alésage distal (68), l'alésage proximal (41) de poignée et l'alésage distal (68) de poignée ayant chacun un diamètre plus grand que la lumière du tube (74) de canule d'accès ;
lors de l'insertion du tube (94) de canule d'administration dans le tube (74) de canule d'accès, ledit au moins un élément (52) de valve pivote dans l'alésage distal (68) de poignée de canule d'accès ;
lors de l'enlèvement du tube (94) de canule d'administration du tube (74) de canule d'accès, l'au moins un élément de valve pivote dans l'alésage proximal (41) de poignée de canule d'accès.

6. Ensemble (20, 140) d'administration selon l'une quelconque des revendications 1 à 5, dans lequel ledit au moins un élément (52, 150) de valve est constitué d'un matériau élastomérique de telle sorte que ledit élément de valve fonctionne comme ledit élément de poussée qui pousse ledit élément de valve jusque dans la position fermée.

7. Ensemble (20, 140) d'administration selon l'une quelconque des revendications 1 à 6, dans lequel ladite valve (48, 146) est un élément plat constitué d'un matériau élastomérique qui a au moins une fente, la fente définissant au moins un clapet (52, 150), ledit clapet étant l'au moins un élément de valve.

8. Ensemble (20, 140) d'administration selon la revendication 7, dans lequel ladite valve (48, 146) est formée avec des fentes multiples de manière à définir plusieurs dits clapets (52, 150) de valve.

9. Ensemble (20, 140) d'administration selon la revendication 7 ou 8, dans lequel ladite valve (150) est en forme de disque.

10. Ensemble (20, 140) d'administration selon la revendication 7, 8 ou 9, dans lequel ladite valve (48, 146) est disposée dans une bague (56, 156) qui entoure circonférentiellement ladite valve.

11. Ensemble (20, 140) d'administration selon l'une quelconque des revendications 1 à 10, dans lequel ladite valve (48, 146) inclut plusieurs dits éléments (52, 150) de valve.

12. Ensemble d'administration selon la revendication 11, dans lequel ladite valve (48, 146) inclut quatre dits éléments (52, 150) de valve.

13. Ensemble (20, 140) d'administration selon l'une quelconque des revendications 1 à 12, dans lequel ladite canule d'administration inclut un raccord (90) formé pour recevoir un raccord (130) de sortie complémentaire d'un dispositif de mélange et d'administration de ciment osseux.

14. Système d'administration de ciment osseux comprenant :
un ensemble (120) de mélange de ciment osseux, l'ensemble de mélange de ciment osseux ayant une première chambre (122) dans laquelle le ciment osseux est mélangé et un raccord (130) de sortie en communication de fluide avec la première chambre à partir duquel le ciment osseux mélangé est administré ;
**caractérisé en ce que** le système comprend en outre :
l'ensemble (20, 140) d'administration selon l'une quelconque des revendications 1 à 13 dans lequel le raccord (90) de canule d'administration est connecté au raccord (130) de sortie de l'ensemble de mélange et d'administration de ciment osseux pour recevoir le ciment osseux mélangé.

15. Système d'administration de ciment osseux selon la revendication 14, dans lequel : l'ensemble (120) de mélange de ciment osseux inclut en outre une deuxième chambre (124) qui est connectée à la première chambre (122) vers laquelle le ciment est fait s'écouler après mélange ; et ledit raccord (130) de sortie de l'ensemble de mélange de ciment osseux est connecté à ladite deuxième chambre (124) pour recevoir le ciment osseux mélangé depuis la deuxième chambre.
